# EUROPEAN PATENT APPLICATION

(11) **EP 2 484 402 A2**
(43) Date of publication of application: **08.08.2012**
(21) Application number: 12154507.3
(22) Date of filing: 08.02.2012
(51) Int. Cl.: A61M 25/00, A61M 39/00

(54) **A venflon component**

(30) Priority: 08.02.2011 DK 201100081
(71) Applicant: Region Nordjylland, Aalborg Sygehus, 9220 Aalborg Ø (DK)
(72) Inventor: Carlsen, Sven, 9200 Aalborg SV (DK)
(74) Representative: Plougmann & Vingtoft A/S

(57) **Abstract**

The application relates to a venflon component (201) which includes a bendable member (202) which makes is possible to redirect the fluid to be delivered by the catheter (101) of the venflon by a bending of an infusion tube which may be part of the venflon or may be connectable to the venflon component. The bendable member is configured by structural shaping to define a minimum bending radius which ensures that the infusion tube is not bent so much that it may kink.

## Description

### FIELD OF THE INVENTION

The invention relates to a component of a venflon, and in particular to a venflon component which is configured to minimize the risk of inducing a kink in the infusion tube.

### BACKGROUND OF THE INVENTION

Intravenous fluid infusion devices or venflons are used for intravenous administration of fluids via a catheter which can be inserted into a vein. The source of fluid is connected to the venflon via an infusion tube. During the attachment to a patient, the infusion tube is normally bent, typically by 180 degrees, for practical reasons. However, the bending may cause the tube to kink or collapse if the bending radius becomes too small. A kink of the tube decreases or blocks the flow of fluid through the tube and, therefore, should be avoided.

WO03/035160 discloses an anti-kinking device, in particular for supporting and stabilising an infusion tubing for fluid infusion in a safety loop, comprising means for receiving and fixing the infusion tubing in connection with a catheter inserted into a superficial vein or the like and connected to an infusion container via the infusion tubing, said device being fixable to a patient in order to relieve the tension of the tubing at the point of insertion. This provides a device for flexible support of an infusion tubing to create a safety loop in a simple and flexible manner. The present invention also relates to such infusion tubing.

Whereas WO03/035160 discloses an anti-kinking device designed to avoid kinking of infusion tubes, the inventor of the present invention has appreciated that improvements are of benefit. Desired improvements may relate to improved handling of devices during arrangement of the venflon, improved non-kinking properties, improved manufacturing capabilities and other improvements. In consequence the inventor has devised the following invention.

### SUMMARY OF THE INVENTION

It would be advantageous to achieve an improved venflon which reduces or eliminates the risk of inducing a kink of an infusion tube. It would also be desirable to improve handling of venflons during insertion and/or fixation of the venflon. In general, the invention preferably seeks to meet one or more of the above mentioned objects, to solve the above mentioned problems, or other problems of the prior art, singly or in any combination.

To better address one or more of these concerns, in a first aspect of the invention a venflon component is presented that comprises
- a catheter for delivering fluid into a vein,
- a connector for receiving an infusion tube,
- a venflon body providing a fluid passageway between the catheter and the connector,
- a bendable tube connecting the connector with the venflon body, where the wall of the tube is shaped to define a minimum bending radius of the tube.

The minimum bending radius of the tube is understood as a bending radius which the tube may obtain by influencing the tube by a normal bending force. Thus, the bending radius is not, or substantially not, exceedable by normal use or by affecting the tube with normal bending forces.

Since the bendable tube is configured so that it has a minimum bending radius which is not, or substantially, not exceedable during normal use, the risk of inducing a kink or a collapse in the bent tube portion may be reduced or even eliminated.

Since the bendable tube is part of the venflon component, handling of the venflon may be eased e.g. during fixation of the venflon. That is, since no separate parts are required to obtain the bending of a tube and for avoiding kinks, handling of the venflon may be improved.

A second aspect of the invention relates to a venflon component, comprising
- a catheter for delivering fluid into a vein,
- a connector for receiving an infusion tube,
- a venflon body providing a fluid passageway between the catheter and the connector,
- a flexible guide for guiding the infusion tube and shaped to define a minimum bending radius of the guided infusion tube.

Similarly to the first aspect, the bending radius is not, or substantially not, exceedable by normal use.

The flexible guide may be shaped to fixate and bend the infusion tube or to support and bend the infusion tube. Since the flexible guide is configured so that it has a minimum bending radius which is not, or substantially, not exceedable during normal use, the risk of inducing a kink or collapse in the bent infusion tube may be reduced or even eliminated by adapting the minimum bending radius of the flexible guide to be larger than the smallest allowable bending radius of the infusion tube.

The flexible guide may be connectable or integrated with the venflon component, so that it is part of the venflon component and, consequently, handling of the venflon component may be eased e.g. during fixation of the venflon.

Common for the venflon components of the first and second aspects is a flexible member shaped to define a minimum bending radius which is not exceedable, where the flexible member is part of a tube of the venflon component connecting the connector with the venflon body or the flexible member is a guide for guiding an infusion tube connectable to the connector. Accordingly, the bendable tube and the flexible guide provides the same technical effects since they are parts of the venflon components and provide means for anti-kinking of a tube extending from the venflon component.

In an embodiment, the bendable tube is compressible and extendable in a longitudinal direction of the bendable tube. The bendable tube may be formed like a bellows or the like, comprising annular corrugations to attain compression and extension capabilities. The tube may be compressed when a needle of another venflon component is inserted through the bendable tube and the catheter for puncturing of a vein. The compressible tube allows the needle to be as short as possible. After the needle has been withdrawn, the tube may be extended and bent as required.

In another embodiment of the present invention, the bendable tube is manufactured in a way so that it is not compressible and extendable in a longitudinal direction of the bendable tube, so as to ensure a longitudinal rigid structure while at the same time being bendable.

However, in a preferred embodiment of the present invention, the tube is both bendable, and at the same time compressible and extendable in a longitudinal direction of the tube, so as to ensure a highly flexible overall structure.

In an embodiment the bendable tube or the bendable portion of the tube has a length which enables reorienting the tube from a straight orientation to a bent orientation where the connector is displaced and rotated by at least 180 degrees relative to the straight orientation. The length of the bendable tube or bendable portion of the tube may be within the range from 1 to 15 centimeters, such as 1 to 10 centimeters, or such as 2 to 8 centimeters, for example 3 to 7 centimeters, such as 4 to 6 centimeters, for example 5 centimeters. The length should be sufficient to enable bending by approximately 180 degrees for a given minimum bending radius of the bendable tube.

In an embodiment an outer surface of the wall of the bendable tube comprises protruding and depressed surface parts being arranged adjacently along the longitudinal direction of the bendable tube, where the longitudinal separation between two neighbor protrusions and the height of the protruding parts relative to the depressed parts in a radial direction defines the minimum bending radius. The protruding and depressed surface parts may be created by embossing features into the outer surface of the tube during manufacturing of the tube, for example subsequent to extrusion of the tube while it is still relatively hot.

In a related embodiment the thickness of the wall of the bendable tube varies as a function of the longitudinal position of the bendable tube so that the variation of the thickness defines the minimum bending radius. Accordingly, the protruding and depressed surface parts may be formed on the outer surface of the tube so that radius of the tube varies as a function of the longitudinal length of the tube, whereas the inner surface is smooth with a constant or substantially constant radius of the lumen of the tube. Advantageously, the inner surface may be smooth to reduce accumulation of e.g. bacteria.

Thus in an embodiment a radius of the lumen of the bendable tube is constant, or substantially constant, along the longitudinal direction of the tube.

In an embodiment an outer surface of a wall of the flexible guide comprises protruding and depressed surface parts being arranged adjacently along the longitudinal direction of the guide, where the longitudinal separation between two neighbor protrusions and the height of the protruding parts relative to the depressed parts in a radial direction defines the minimum bending radius.

Thus, similarly to the protruding and depressed surface parts of the bendable tube, the protruding and depressed surface parts of the flexible guide features a minimum bending radius of the guide which is not easily exceedable by normal use.

In an embodiment the flexible guide comprises a concave member shaped to at least partly surround a longitudinal section of the infusion tube. The curvature of the concave shaped part may be selected to match the outer curvature of the infusion tube to enable holding of the infusion tube or just support of the infusion tube. Thus, the concave shaped member may be smooth on the side for holding or supporting the infusion tube, and provided with protruding and depressed surface parts on an opposite side.

A third aspect relates to venflon assembly, comprising
- a venflon component according to the first or second aspect, and
- venflon component with a needle for puncturing a vein, where the needle is insertable into the catheter.

The venflon assembly may comprise other components such as a plug and a needle protection cap.

It is within the scope of the present invention, that the venflon components according to the first, second and third aspects of the invention, may be manufactured in any suitable material known to the person skilled in the art such as, but not limited to, any kind of plastic material, rubber, ceramics and any type of metal, including any kind of alloy compositions.

In one embodiment, the venflon components according to the first, second and third aspects of the invention are manufactured in a plastic material such as, but not limited to, nylon, polyethylene and/or polypropylene, and/or a thermoplastic material such as acrylonitrile-butadiene styrene (ABS).

In another embodiment, the venflon components according to the first, second and third aspects of the invention are manufactured in a metal material such as, but not limited to, stainless steel and/or aluminum.

It is also within the scope of the present invention, that the individual members of the venflon components and venflon assemblies according to the first, second and third aspect of the invention in one embodiment may be manufactured as multiple individual parts, whereafter the individual member parts are connected, and assembled to form the ready-to-use venflon component and/or venflon assembly.

In another embodiment, the individual members of the venflon components and venflon assemblies according to the first, second and third aspect of the invention may be manufactured as one single continuous part, which then forms the ready-to-use venflon component and/or venflon assembly.

A fourth aspect of the invention relates to a method for arranging a venflon component, the venflon component comprising a catheter for delivering fluid into a vein, a connector for receiving an infusion tube, a venflon body providing a fluid passageway between the catheter and the connector, the method comprising
- using a flexible member shaped to define a minimum bending radius which is not exceedable by normal use where the flexible member is one of:
- a bendable tube connecting the connector with the venflon body, where the wall of the tube is shaped to define the minimum bending radius,
- a flexible guide for guiding the infusion tube and shaped to define the minimum bending radius, or
- a flexible connecting tube connectable in one end with the connector and in another end with the infusion tube, where the wall of a section of the connection tube is shaped to define the minimum bending radius,
for obtaining a bent fluid passageway defined by the bendable tube, the flexible guide or the flexible connection tube.

According to this aspect, instead of using a flexible guide or bendable tube of the venflon component, a separate flexible and bendable connecting tube connected to the venflon may be used to obtain a bending of the tube supplying fluids to the catheter.

In summary the invention relates to a venflon component which includes a bendable member which makes is possible to redirect the fluid to be delivered by the catheter of the venflon by a bending of an infusion tube which may be part of the venflon or may be connectable to the venflon component. The bendable member is configured by structural shaping to define a minimum bending radius which ensures that the infusion tube is not bent so much that it may kink.

In general the various aspects of the invention may be combined and coupled in any way possible within the scope of the invention. These and other aspects, features and/or advantages of the invention will be apparent from and elucidated with reference to the embodiments described hereinafter.

### BRIEF DESCRIPTION OF THE DRAWINGS

Embodiments of the invention will be described, by way of example only, with reference to the drawings, in which
Fig. 1 shows a known venflon,
Fig. 2 shows a venflon with a bendable tube,
Fig. 3A shows a tube with a profiled outer surface and a smooth inner surface,
Fig. 3B shows the difference between a tube with a smooth inner surface and a profiled inner surface,
Fig. 4A shows a connection tube having a bendable section connected to a venflon,
Fig. 4B shows a connection tube in a bent configuration, and
Fig. 5 shows a venflon with a flexible guide for an infusion tube.

### DETAILED DESCRIPTION OF EMBODIMENTS

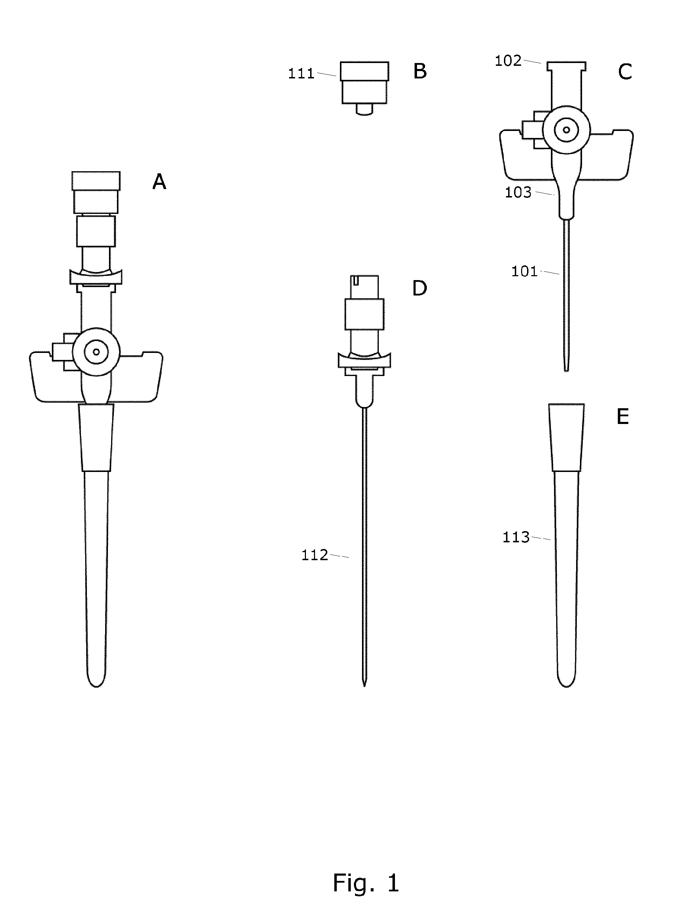

Fig. 1 illustrates a known intravenous fluid infusion device, referred to in this description as a venflon. Illustration A shows the venflon assembled from components B-E. Illustration B shows a plug 111. Illustration C shows a component which comprises a catheter 101 for delivering fluid into a vein, a connector 102 for receiving an infusion tube and a venflon body 103 providing a fluid passageway being at least part of the entire passageway connecting the catheter 101 and the connector 102. Illustration D shows a component which comprises a needle 112 for puncturing the vein. Component E shows a protection cap for the needle which is removed before use.

During use of the venflon the needle which protrudes from the catheter is used to puncture the vein. Subsequently, the catheter is pushed into the vein through the hole made by the needle. After this the needle is withdrawn so that only the component shown in illustration C is left. The plug 111 may be connected to the connector 102 or an infusion tube may be connected to the connector 102.

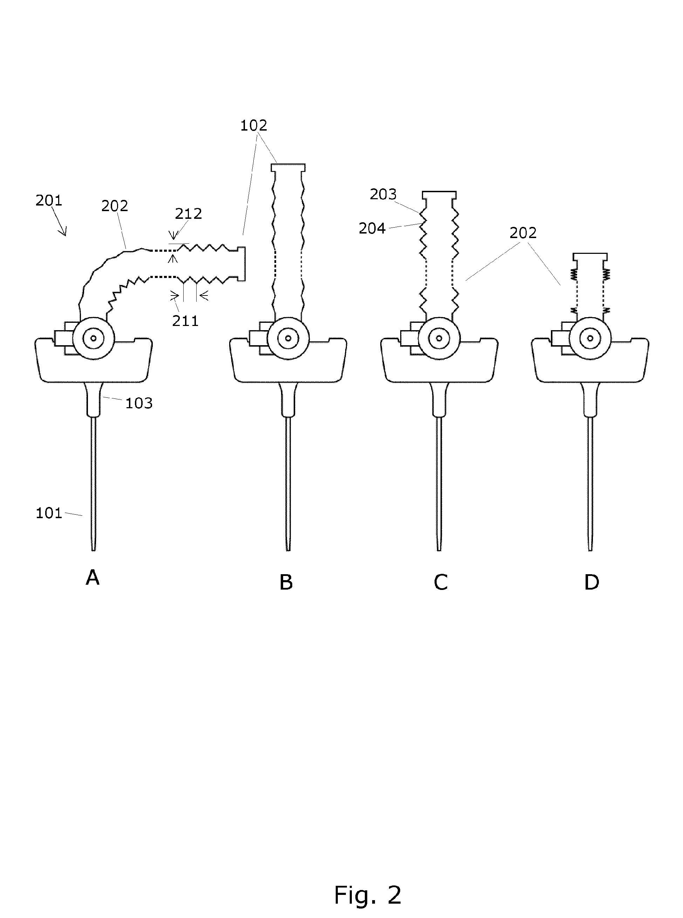

Fig. 2 illustrates an embodiment of the invention. Illustrations A-D show different states of a venflon component 201 which has the same function as the venflon component shown in illustration C in Fig. 1. Accordingly, the venflon component 201 comprises a catheter 101, a connector 102 and a venflon body 103 which are equivalent to the corresponding parts with the same reference numbers of Fig. 1. The venflon component 201 differs from the component in illustration C in Fig. 1 by having a bendable tube 202 connecting the connector 102 with the venflon body 103.

The wall of the tube is shaped to define a minimum bending radius of the tube which is not, or substantially not, exceedable by normal use. The minimum bending radius is achievable by bending the tube 202.

Illustration A in Fig. 2 shows a 90 degrees bending of the tube, illustration B shows a state where the tube is maximally stretched, illustration C shows an intermediate stretch of the tube and illustration D shows a state where the tube is maximally compressed. The tube may be configured to be bendable up to e.g. 180, 225 degrees or other degrees. It may be beneficial that the tube is compressible as shown in Fig. 2(D) in order to minimize the length of the needle which needs to extend trough the tube 202 and the catheter 101 so as to enable puncturing of a vein by the needle protruding from the end of the catheter.

After puncturing of the vein, the needle component of the venflon is withdrawn, the catheter is positioned in the vein and secured e.g. to the hand of a patient. Then the bendable tube 202 may be stretched and/or bent e.g. to form a U-Ioop so that an infusion tube can be connected to the connector 102. Due to the bending of the tube, the infusion tube can be positioned in a way which is most convenient for the patient and/or for connection with clinical devices such as a fluid container containing fluid to be guided to the venflon. Furthermore, the bending of the tube may provide a safety loop which minimizes the risk for repositioning of the catheter due to inadvertent pulling of the infusion tube.

Whereas a normal infusion tube may kink and collapse if it is bent so that the bending radius becomes smaller than a threshold, the bendable tube has a minimal non-exceedable bending radius which is selected to be larger than a critical bending radius where the tube may kink.

Due to the shaping of the bendable tube it may be bendable to a bending radius which is smaller than the bending radius of a normal infusion tube which is normally bent in order to obtain the most convenient positioning.

The shaping of the tube 202 for defining a minimum bending radius may be embodied by a bellows shaped tube comprising annular corrugations as shown in Fig. 2. The bellows shaped tube is formed so that protruding 203 and depressed 204 surface parts (i.e. ridges and valleys) are arranged adjacently along the longitudinal direction of the bendable tube. The longitudinal separation 211 between two neighbor protrusions and the height 212 of the protruding parts relative to the depressed parts in a radial direction defines the minimum bending radius. That is, the larger the separation 211 and/or the lower the height 212 are the smaller bending radius is defined. Thus, as adjacent protruding parts 203 come closer along the compressed side of the tube and adjacent protruding parts 203 come farther apart along the stretched side of the tube due to bending, the bending stiffness increases until a given minimum bending radius where the tube is not, or substantially not, further bendable. The user may able to bend the tube beyond the minimum bending radius, but only by use of an increased force or inappropriate handling of the tube.

Additionally since adjacent wall sections, i.e. adjacent wall sections connecting a protrusion 203 with a depression 204, makes an angle to each other which is smaller than 180 degrees, the bellows shaping comprising annular corrugations shown in Fig. 2 allows compression and extension of the tube since the wall of the tube is able to collapse or extend in a longitudinal direction of the tube.

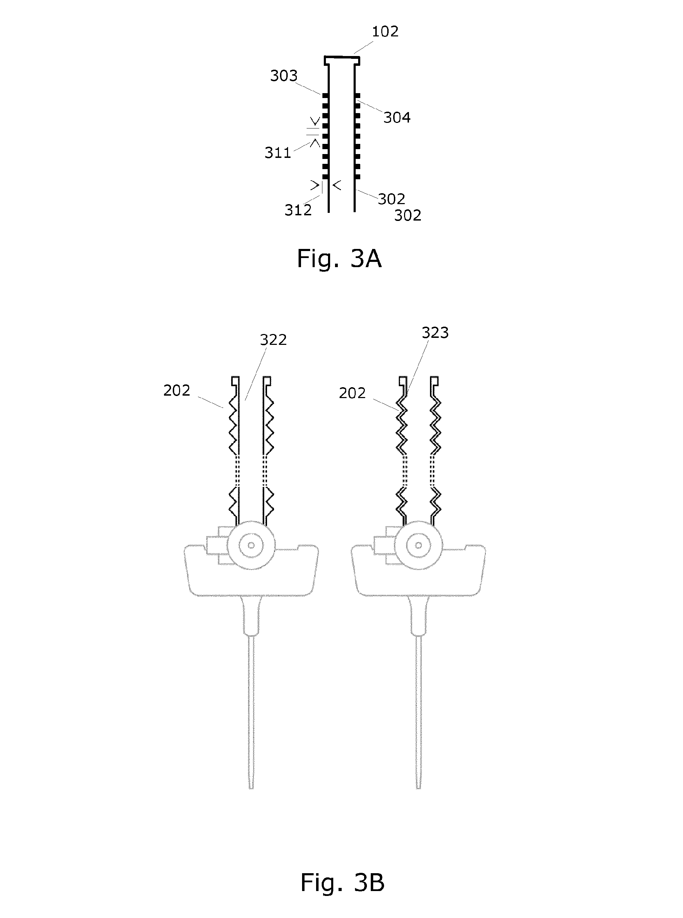

Fig. 3A shows an alternative shaping defining a minimum bending radius of the bendable tube 302 where the shaping is embodied by providing a flexible tube with protrusions 303 extending radially away from the center of the bendable tube 302 and forming thickenings of the wall of the tube. The protrusions 303 may fully or partly circumscribe the tube. Two separated and adjacent protrusions 303 form a valley or depression 304. Again the longitudinal separation 311 between two neighbor protrusions 303 and the height 312 of the protruding parts relative to the depressed parts in a radial direction defines the minimum bending radius. That is, as the tube 302 is bent adjacent protrusions will eventually contact so that the tube cannot be bent further and, thus, the protrusions define the minimal bending radius.

Fig. 3B, left illustration, shows a bendable tube where the inner surface 322 of the tube has a smooth surface **characterized in that** the radius of the cylindrical lumen of the bendable tube is constant, or substantially constant, along the longitudinal direction of the tube. In comparison, the right illustration shows a tube where the radius of the lumen varies as a function of the longitudinal position of the tube.

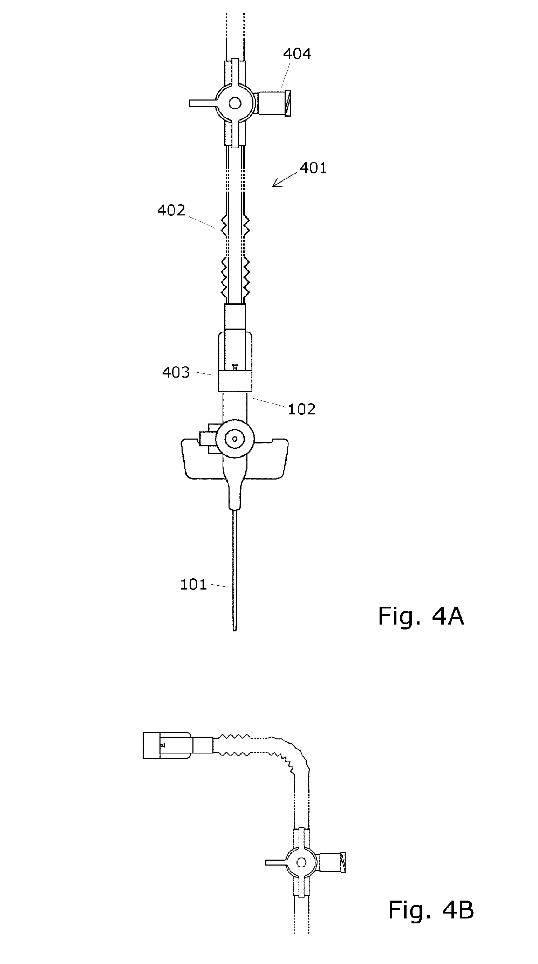

Fig. 4A shows an aspect of the invention where the venflon component having a catheter 101 is not provided with a bendable tube. Instead an infusion tube or part of an infusion tube which is connectable with the connector 102 of the venflon component via a connector 403 of the infusion tube is provided with a bendable tube portion 402 configured equivalently to the bendable tube of the venflon component in order to define a minimum bending radius of the infusion tube. Thus, by use of a bendable infusion tube, i.e. a flexible connecting tube 401 which is connectable in one end with the connector 102 of the venflon component and in another end with an infusion tube comprising another connector 404, a bent fluid passageway may be made by the bendable tube portion 402. Fig. 4B shows the flexible connecting tube 401 in a bent configuration.

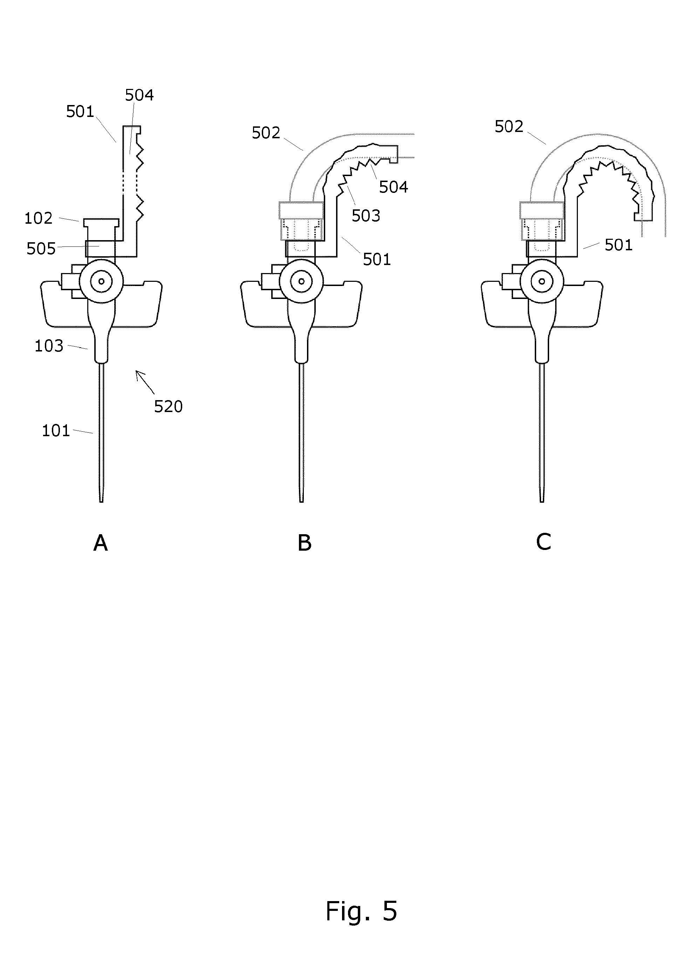

Figs. 5A-C show a venflon component 520 comprising a flexible bendable guide 501 for guiding a normal infusion tube 502. Equivalently to the bendable tube 202 or tube portion 402 the bendable guide 501 is shaped to define a minimum bending radius of the guided infusion tube which is not exceedable by normal use.

The minimum bending radius of the flexible guide may be achieved by forming the outer surface of the wall of the flexible guide 501 with protruding 503 and depressed 504 surface parts being arranged adjacently along the longitudinal direction of the guide, where the longitudinal separation between two neighbor protrusions and the height of the protruding parts relative to the depressed parts in a radial direction defines the minimum bending radius.

Thus, the guide is bendable until adjacent protruding surface parts or ridges of the guide eventually contacts or come close to each other. Accordingly the minimum bending radius is defined by the height of protruding parts and the distance between protruding parts. The flexible guide may be formed by nylon, rubber or similar plastic materials with flexible properties, as well as any type of metal, including stainless steel and aluminum.

The flexible guide may comprise a concave shaped part such as a jacket shaped to at least partly surround a longitudinal section of the infusion tube. Thus, the concave shaped part has an inner surface, preferably with a smooth surface, for supporting a section of the infusion tube. The concavely shaped part may surround the infusion tube by less than 180 degrees of an arc of the periphery of the tube, or greater than 180 degrees. The concave shaped part may be shaped with an inner diameter which is slightly less than the outer diameter of the infusion tube to obtain a fixture of the infusion tube, or the inner diameter may be greater than the outer diameter of the infusion tube to provide only a support for the tube.

In an embodiment the flexible guide may be a tube shaped guide such as a flexible and bendable hose which completely surrounds a section of the infusion tube.

The flexible guide 501 may be an integrate part of the venflon component 520 or the flexible guide 501 may comprise a connector 505 connectable with a part of the venflon component 520, e.g. the neck of the connector 102.

Fig. 5A shows the venflon component 520 without the infusion tube, and Fig. 5B-C shows the venflon with the infusion tube connected via the connector 102 and bent by respectively 90 and 180 degrees in Fig. 5B and 5C. The bending of the infusion tube 502 and the flexible guide may be achieved by a tension in the infusion tube, e.g. in a direction perpendicular to the longitudinal direction of the needle 101.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

Any reference signs in the claims should not be construed as limiting the scope.

## Claims

1. A venflon component (201), comprising
- a catheter (101) for delivering fluid into a vein,
- a connector (102) for receiving an infusion tube,
- a venflon body (103) providing a fluid passageway between the catheter and the connector,
- a bendable tube (202) connecting the connector with the venflon body, where the wall of the tube is shaped to define a minimum bending radius of the tube.

2. A venflon component (520), comprising
- a catheter (101) for delivering fluid into a vein,
- a connector (102) for receiving an infusion tube,
- a venflon body (103) providing a fluid passageway between the catheter and the connector,
- a flexible guide (501) for guiding the infusion tube and shaped to define a minimum bending radius of the guided infusion tube.

3. A venflon component according to claim 1, where the bendable tube is compressible and extendable in a longitudinal direction of the bendable tube.

4. A venflon component according to claim 1, where the bendable tube has a length which enables reorienting the tube from a straight orientation to a bent orientation where the connector is displaced and rotated by at least 180 degrees relative to the straight orientation.

5. A venflon component according to claim 1, where an outer surface of the wall of the bendable tube comprises protruding (203, 303) and depressed (204, 304) surface parts being arranged adjacently along the longitudinal direction of the bendable tube, where the longitudinal separation between two neighbor protrusions and the height of the protruding parts relative to the depressed parts in a radial direction defines the minimum bending radius.

6. A venflon component according to claim 1, where a radius of the lumen of the bendable tube is constant, or substantially constant, along the longitudinal direction of the tube.

7. A venflon component according to claim 2, where an outer surface of the wall of flexible guide comprises protruding (503) and depressed (504) surface parts being arranged adjacently along the longitudinal direction of the guide, where the longitudinal separation between two neighbor protrusions and the height of the protruding parts relative to the depressed parts in a radial direction defines the minimum bending radius.

8. A venflon component according to claim 2, where the flexible guide comprises a concave member shaped to at least partly surround a longitudinal section of the infusion tube.

9. A venflon, comprising
- a venflon component according to claim 1 or 2, and
- venflon component with a needle (112) for puncturing a vein, where the needle is insertable into the catheter.

10. A method for arranging a venflon component, the venflon component comprising a catheter for delivering fluid into a vein, a connector for receiving an infusion tube, and a venflon body providing a fluid passageway between the catheter and the connector, the method comprising
- using a flexible member shaped to define a minimum bending radius which is not exceedable by normal use, where the flexible member is one of:
- a bendable tube connecting the connector with the venflon body, where the wall of the tube is shaped to define the minimum bending radius,
- a flexible guide for guiding the infusion tube and shaped to define the minimum bending radius, or
- a flexible connecting tube (401) connectable in one end with the connector and in another end with the infusion tube, where the wall of a section of the connection tube is shaped to define the minimum bending radius,
for obtaining a bent fluid passageway defined by the bendable tube, the flexible guide or the flexible connection tube.
